# EUROPEAN PATENT APPLICATION

(11) **EP 4 706 781 A1**
(43) Date of publication of application: **11.03.2026**
(21) Application number: 25197863.1
(22) Date of filing: 25.08.2025
(51) Int. Cl.: A62B 7/14, A62B 9/00

(54) **APPARATUS FOR MONITORING OXYGEN PARTIAL PRESSURE AND ALERTING A USER OF IMMINENT RISK OF HYPOXIA**

(30) Priority: 10.09.2024 SE 2400100
(71) Applicant: Triton Lynx AB, 439 52 Åsa (SE)
(72) Inventor: Lewis-Lück, Rebecca, 43952 Åsa (SE); Lewis-Olsson, Magnus, 43952 Åsa (SE); Loncar, Mario, 17839 Ekerö (SE)
(74) Representative: Heimdal, Pär

(57) **Abstract**

An apparatus (130) for monitoring oxygen partial pressure and alerting a user of imminent risk of hypoxia. The apparatus includes a first attachment mechanism (131) for a breathing gas inlet of a respiratory mask (120) and a second attachment mechanism (132) for attachment to a breathing gas tubing (140). A sensor (310) measures the oxygen partial pressure in the tubing. A vibration actuator (335) provides a tactile alert causing vibrations on the respiratory mask (120) when activated. A controller (320), connected to the sensor (310) and vibration actuator (335), obtains and compares pressure measurements with a warning level, triggering an alert when the pressure is low. A power supply unit (340) powers the sensor (310), vibration actuator (335), and controller (320). The sensor (310), vibration actuator (335), controller (320), and power supply unit (340) are housed in one unit, attachable to the respiratory mask (120) and breathing gas tubing (140).

## Description

### TECHNICAL FIELD

This document relates to aviation safety during high altitude operations. In particular, this document relates to an apparatus for monitoring oxygen partial pressure in the inhaled breathing gas and alerting a user of imminent risk of hypoxia when the oxygen partial pressure is lower than a pressure warning level.

### BACKGROUND

Aviation safety during high altitude operations involves ensuring that pilots receive adequate oxygen levels to prevent hypoxia, i.e. low concentration of oxygen in the blood. This condition is particularly dangerous for pilots as it can impair cognitive functions and physical coordination, leading to potentially fatal consequences.

In military aviation, the pilot is supplied with oxygen enriched breathing gas either supplied from onboard storage of oxygen or, as is the case in most modern fighter aircraft, a so called On-Board-Oxygen-Generating-System (OBOGS) to maintain adequate oxygen levels during high altitude flights. These systems provide breathing gas to the pilot's respiratory mask, thereby extending the operational altitude range of the aircraft.

Despite the use of supplemental oxygen systems, pilots may still experience hypoxia due to reduced oxygen levels at very high altitudes. Hypoxia can manifest through various symptoms, including cognitive performance failure, fatigue, dizziness, tunnel vision, poor coordination, headache, stress reactions, and confusion. In severe cases, hypoxia may cause the pilot to lose consciousness, which can result in catastrophic incidents if the pilot cannot be revived promptly.

Fighter pilots undergo specialized training to recognize the symptoms of hypoxia. This training typically involves exposure to hypoxic conditions in controlled environments, to help pilots identify the early signs of oxygen deprivation. These signs are to some extent individual. However, this self-awareness training is not always sufficient, and pilots may fail to detect hypoxia in time, leading to severe incidents. Some of these symptoms or signs of hypoxia may also coincide or be interpreted as reaction to high g-force, or possibly dehydration/ stress/ exhaustion which may lead the pilot to an incorrect conclusion.

It is desired to find a technical solution for preventing hypoxia of pilots, in particular fighter pilots. This solution should enhance pilot safety by ensuring that hypoxia is detected and addressed promptly, thereby increasing aviation safety on high altitude.

### SUMMARY

It is therefore an objective to prevent a user experiencing hypoxia at higher altitudes. The user may be a pilot and/ or other personnel onboard a civilian or military aircraft, parachuters, special forces operators, jumpmasters etc.

According to one aspect, an apparatus is provided, for monitoring the level of oxygen partial pressure and alerting a user of imminent risk of hypoxia. The apparatus comprises a first attachment mechanism configured to attach the apparatus to a breathing gas inlet of a respiratory mask of the user. Also, the apparatus comprises a second attachment mechanism configured to attach the apparatus to a breathing gas tubing providing breathing gas/ oxygen-enriched air from an onboard oxygen generation system to the respiratory mask.

The first and/ or second attachment mechanisms may comprise a threaded attachment mechanism in some embodiments. However, in other embodiments, the respective attachment mechanisms may comprise a barbed connector, snap or click connector, a twist lock mechanism, a clamped connection, a rubber grommet, a magnetic connector, or similar connection mechanisms, for example.

The apparatus comprises a sensor configured to measure the oxygen partial pressure in the breathing gas tubing. The sensor may measure the oxygen partial pressure continuously at a regular time interval, for example every second, every five seconds, etc. Alternatively, the sensor may measure the oxygen partial pressure at an irregular time interval or possibly upon request.

The apparatus also comprises a vibration actuator, situated in a position abutting a part of the respiratory mask or in close vicinity to the respiratory mask. The vibration actuator is configured to provide a tactile alert causing vibrations on the respiratory mask when activated.

"Close vicinity" in the current context is to be regarded as a distance over which the vibration actuator is able to directly or indirectly cause vibrations on the respiratory mask, for example some millimeters, or some few centimeters.

The vibration actuator may for example comprise a vibration motor such as a coin vibration motor or a cylindrical vibration motor. Alternatively, the vibration actuator may comprise a Linear Resonant Actuator or similar vibration generating structure.

The apparatus in addition comprises a controller, communicatively connected to the sensor and the the vibration actuator, configured to obtain a measurement of the oxygen partial pressure from the sensor; compare the obtained measurement with a pressure warning level; and trigger an alert via the vibration actuator when the oxygen partial pressure is lower than the pressure warning level.

The controller may obtain measurements of the oxygen partial pressure from the sensor continuously, at a regular time interval, or upon request. The measurement request may be triggered by a timer, for example every second; or alternatively by the user for example before take-off for checking functionality of the apparatus.

The apparatus also comprises a power supply unit configured to power the sensor, the vibration actuator, the controller and possibly other entities comprised in the apparatus.

The power supply unit may for example comprise an internal battery, which eliminates the need for external power sources and enhances the independence and reliability of the apparatus. The battery's inclusion within the single unit apparatus further contributes to the apparatus's ease of use. This design ensures that the apparatus can function autonomously, providing a reliable safety mechanism for pilots/ users onboard an aircraft without interfering with other onboard systems or any other pilot equipment.

Optionally however, the power supply unit may also, or alternatively comprise a power supply enabler for providing the apparatus with electric power support from an apparatus-external source.

The sensor, the vibration actuator, the controller, and the power supply unit are maintained in one single unit, separate from, although attachable to, the respiratory mask and the breathing gas tubing via the respective attachment mechanisms.

The apparatus provides a comprehensive solution for monitoring oxygen partial pressure and alerting the user of imminent risk of hypoxia by integrating all necessary components into one single unit, without requiring additional cables or external power sources. By not involving more sensors, entities, measurements or calculations than necessary, the size and weight of the apparatus is kept small. Thereby visibility and/ or mobility of the user is not affected.

During operation, the working environment onboard for example a fighter plane is subject to enhanced gravitational forces. As the apparatus is compact and lightweight, influence of gravitational forces during operation is minimised.

**The** inclusion of the oxygen partial pressure sensor allows for real-time monitoring of oxygen partial pressure in the inlet breathing gas tubing, ensuring that any drop in oxygen partial pressure is detected promptly. By measuring the oxygen partial pressure in the inlet breathing gas tubing, instead of for example measuring various physiological parameters of the user/ pilot, hypoxia of the user could be predicted and thereby avoided before actually occurring, rather than detected as an already existing condition of the user, thereby enhancing safety.

Also, by only utilising the oxygen partial pressure in the inlet breathing gas tubing and no other parameter for triggering the pressure warning alert, complexity of the provided solution is minimised. Computational efforts are thereby also minimised, enhancing robustness of the provided solution.

**The** single-unit design also simplifies installation and maintenance, and/ or replacement, as it can be easily attached to the respiratory mask and the breathing gas tubing via the respective attachment mechanisms, possibly even without any tools. Also, the apparatus could be installed without making any changes at all in existing equipment such as the respiratory mask, breathing gas tubing and/ or helmet.

The tactile alert of the vibration actuator acting directly on the respiratory mask is particularly effective in the noisy and stressful environment of a fighter cockpit and other aircrafts, where visual and/ or auditory alerts may be less noticeable, or even pass completely undetected by the user.

According to another aspect, the apparatus may comprise a visual output device communicatively connected to the controller, wherein the visual output device is configured to provide a visual alert when activated. The controller may be configured to trigger the alert also via the visual output device when the oxygen partial pressure is lower than the warning level.

The visual output device may comprise e.g. a display, a diode, or a set of diodes situated on an exterior of the apparatus.

The disclosed arrangement combining tactile and visual alerts significantly improves pilot safety by providing multiple opportunities to recognize and respond to hypoxia. The combination of tactile and visual alerts leverages different sensory pathways, making the alert system more robust and reliable in various operational conditions.

According to another aspect, the controller may be configured to compare the obtained measurement of the oxygen partial pressure in the breathing gas inlet/ tubing with a plurality of pressure warning levels and trigger different types of alerts, based on the outcome of the comparison.

By configuring the controller to compare the obtained measurement with a plurality of pressure warning levels and trigger different types of alerts based on the outcome of the comparison, the apparatus can provide a more nuanced and graduated response to varying levels of hypoxia risk. This allows for a more tailored alerting that can escalate in intensity as the oxygen partial pressure decreases, thereby providing the user with timely and appropriate warnings that can help prevent the onset of severe hypoxia.

This multi-level alert solution enhances the pilot's ability to respond to hypoxia in a timely manner, as different alert types can be used to indicate different levels of urgency. For example, a brief and gentle tactile alert/ visual output can be used for initial warnings, merely predicting an increased risk, while more intense alerts, such as repeated forceful vibrations in combination with blinking visual alerts can be used for more severe conditions. In alternative embodiments, another type of sensory modality may be activated by a distinct sensory organ stimulus, for example an olfactory alert.

In yet other embodiments, different colours of the visual output may carry and communicate different oxygen partial pressure level intervals, for example green: satisfactory oxygen partial pressure; yellow: oxygen partial pressure is falling/ approaching the warning level; red: the oxygen partial pressure is lower than the warning level. In yet some embodiments, one ignited diode may indicate satisfactory oxygen partial pressure; two ignited diodes may indicate that the oxygen partial pressure is falling/ approaching the warning level; three ignited diodes may indicate that the oxygen partial pressure is lower than the warning level.

This graduated multi-level alert solution ensures that the user is continuously informed about the oxygen partial pressure levels, in particular when the oxygen partial pressure falls to a level representing an increased risk of hypoxia, thereby improving overall safety during high-altitude operations.

According to another aspect, the controller may be configured to provide escalating alerts, such as e.g. visual and/ or tactical alerts, in response to detection of decreasing oxygen partial pressure levels.

Thereby, a multi-tiered alert solution is achieved that can escalate in response to decreasing oxygen levels. This ensures that the user receives timely and appropriate alerts, and/ or eliminates or at least reduces the risk that the alerts pass the user unattended, thereby enhancing the likelihood of recognizing and addressing imminent risk of hypoxia.

According to an aspect, the escalating alerts may comprise an increase in vibration amplitude and/ or vibration intensity of the vibration actuator and/ or variation in emitted colour and/ or increased blinking intensity of the visual output device.

This arrangement significantly improves user safety by providing multiple opportunities to recognize and respond to an emerging risk of hypoxia. The optional combination of tactile and visual alerts leverages different sensory pathways, making the alert system more robust and reliable in various operational conditions.

According to an aspect, any pressure warning level may be adaptable to an individual hypoxia sensibility of the user.

By allowing the pressure warning levels to be adaptable to an individual user's hypoxia sensitivity, the apparatus can provide a more personalized and effective alert system. This customization ensures that the alerts are triggered at the most appropriate times for each user, taking into account individual differences in lung capacity, health status, age, and other factors. This adaptability enhances the overall safety and effectiveness of the hypoxia alert system, as it can be fine-tuned to meet the specific needs of each user, thereby reducing the risk of hypoxia-related incidents.

According to yet another aspect, the power supply unit may comprise a rechargeable battery.

By incorporating a rechargeable battery, the apparatus ensures continuous operation without requiring frequent battery replacements, thereby enhancing the reliability and convenience of the device. This feature is particularly advantageous in a fighter plane, which may have to land on a war-time airfield (basically a highway strip with a refuelling capacity, serving as an auxiliary military air base), or an airfield in another country, on which supplement batteries of the right type cannot be expected to always be available.

However, recharging the battery for example via a Universal Serial Bus (USB) port may be commonly available, for example from a cell phone charger, a computer, a portable power bank, a solar cell driven charger device, etc.

The rechargeable battery also contributes to the sustainability of the apparatus by reducing waste associated with disposable batteries. The rechargeable battery allows for the apparatus to be more cost-effective over time, as the need for purchasing and replacing disposable batteries, and for keeping supply storages of fresh batteries at relevant airfields are eliminated.

According to another aspect, the power supply unit may comprise a wirelessly rechargeable battery.

Incorporating a wirelessly rechargeable battery into the apparatus enhances its operational convenience and reliability. This feature eliminates the need for physical connectors or ports, which can be prone to wear and tear or damage, for example due to e.g. dust or moisture, especially in the demanding environment of a fighter cockpit.

The wireless recharging capability ensures that the power supply unit/ battery can be recharged effortlessly, reducing downtime and maintenance efforts, for example by charging the battery when the helmet and respiratory mask are placed on their storage room between missions. Thereby, the battery is recharged automatically and effortlessly, without needing to connect any cables. This can be particularly advantageous in scenarios where quick turnaround times are essential, such as during rapid deployment or between training sessions.

According to another aspect, the apparatus may comprise a memory configured to store measurement data during operation. The apparatus may also comprise a data logging interface configured to export collected measurement data from the memory, for post-operation analysis, for example in an aircraft-external computational resource.

Integrating a data logging interface into the apparatus allows for the collection and storage of measurement data from the sensor. This feature enables post-flight analysis of oxygen levels, which can be crucial for understanding the conditions that led to hypoxia incidents and for improving future flight safety protocols.

By providing a means to collect and analyse data, the apparatus enhances its utility beyond real-time monitoring and alerting. The recorded data can be used for long-term studies on pilot health and performance, contributing to the development of more effective hypoxia prevention strategies and technologies.

According to an aspect, the apparatus may comprise an ambient pressure sensor configured to measure ambient pressure and/ or an accelerometer configured to measure acceleration. The measurement data stored by the memory and exported for post-operation analysis may comprise the measured ambient pressure i.e. the cabin pressure and/ or acceleration.

By providing a means to collect and analyse data, the apparatus enhances its utility beyond real-time monitoring and alerting. The recorded data can be used for long-term studies on user health and performance, contributing to the development of more effective hypoxia prevention strategies and technologies.

According to another aspect, the controller may be configured to run a self-test program configured to confirm correct apparatus functionality and power supply status, during startup of the device and/ or upon manual request by the user.

By configuring the controller to run a self-test program upon manual request by the user, the apparatus ensures that its functionality and power supply status can be verified before use. This feature enhances the reliability and safety of the device, as it allows the user to confirm that the apparatus is operating correctly, and that the power supply unit is operational/ sufficiently charged before embarking on a mission. This pre-flight check can prevent potential failures during critical operations, thereby improving overall flight safety.

The self-test program provides a convenient and efficient way for users to ensure the apparatus is in optimal working condition without requiring additional diagnostic tools or external systems. This capability is particularly valuable in the high-stress and time-sensitive environment of military aviation, where quick and reliable equipment checks are essential.

According to an alternative aspect, the apparatus may comprise an olfactory emitter communicatively connected to the controller. The olfactory emitter may be configured to provide an olfactory alert to the respiratory mask when activated. The controller may be configured to trigger the alert also via the olfactory emitter when the oxygen partial pressure is lower than the warning level.

The optional olfactory emitter provides yet an additional sensory modality for alerting the user, which is particularly useful in the challenging and noisy environment of a fighter cockpit.

This olfactory alert can be more noticeable than visual or auditory alerts, which may be overlooked or drowned out by the surrounding noise and activity. The olfactory alert may comprise a scent and/ or a restorative substance.

In case the olfactory alert comprises a restorative substance such as smelling salt or ammonia, the olfactory alert may provide a boost in alertness during an early stage of hypoxia, as the restorative substance irritates nasal and lung membranes, thereby triggering an inhalation reflex that momentarily improves oxygen intake of the user. The user is thereby recovered from the early stage of hypoxia and is given opportunity to manually release emergency oxygen.

Other advantages and additional novel features will become apparent from the subsequent detailed description.

### FIGURES

Embodiments of the invention will now be described in further detail with reference to the accompanying figures, in which:
Figure 1 illustrates a pilot wearing a respiratory mask with an attached apparatus for monitoring oxygen partial pressure according to an embodiment.
Figure 2 illustrates a structure with attachment mechanisms for connecting the apparatus to a breathing gas inlet of the respiratory mask and the oxygen tubing, respectively.
Figure 3 is a schematic block diagram illustrating the components and connections of the apparatus for monitoring oxygen partial pressure and alerting the user/ pilot of imminent risk of hypoxia according to an embodiment.

### DETAILED DESCRIPTION

Embodiments of the invention described herein are defined as an apparatus for monitoring level of oxygen partial pressure and alerting a user of imminent risk of hypoxia, which may be put into practice in the embodiments described below. These embodiments may, however, be exemplified and realised in many different forms and are not to be limited to the examples set forth herein; rather, these illustrative examples of embodiments are provided so that this disclosure will be thorough and complete.

Still other objects and features may become apparent from the following detailed description, considered in conjunction with the accompanying drawings. It is to be understood, however, that the drawings are designed solely for purposes of illustration and not as a definition of the limits of the herein disclosed embodiments, for which reference is to be made to the appended claims. Further, the drawings are not necessarily drawn to scale and, unless otherwise indicated, they are merely intended to conceptually illustrate the structures and procedures described herein.

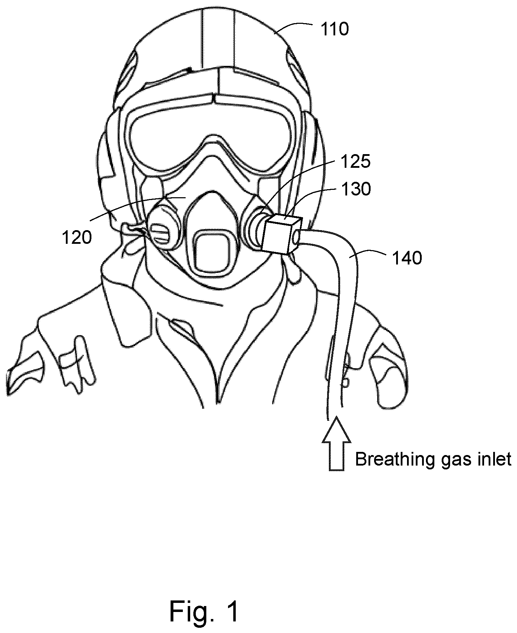

Figure 1 illustrates a scenario with a pilot wearing a pilot helmet 110. A respiratory mask 120 is attached to the pilot helmet 110. A breathing gas tubing 140 provides breathing gas, for example oxygen-enriched air from an oxygen source, such as for example an Onboard Oxygen Generation System (OBOGS) or similar entity. The purpose of the provided breathing gas/ oxygen-enriched air is to support the pilot/ user with adequate oxygen levels during high-altitude operations.

The pilot helmet 110 is designed to protect the pilot's head and support the respiratory mask 120. The pilot helmet 110 ensures that the respiratory mask 120 remains securely in place during flight operations.

The respiratory mask 120 covers the user's/ pilot's nose and mouth, providing a sealed environment for delivering breathing gas. The respiratory mask 120 connects to an apparatus 130, which monitors the oxygen partial pressure in the inhaled air in the breathing gas tubing 140, which is passing the apparatus 130.

The apparatus 130 is connectable to a breathing gas inlet 125 of the respiratory mask 120 at a first end section of the apparatus 130, and to the breathing gas tubing 140 at a second end section of the apparatus 130. Thus, breathing gas of the breathing gas tubing 140 is passing through the apparatus 130 immediately before reaching the respiratory mask 120. An advantage with placing the apparatus 130 at the breathing gas inlet 125 of the respiratory mask 120, i.e. at the end of the breathing gas tubing 140. Any oxygen partial pressure caused by leakage of the breathing gas tubing 140 and/ or any system components prior to the breathing gas tubing 140 is thereby detected by the apparatus 130.

The apparatus 130 is designed to continuously monitor the oxygen partial pressure in the breathing gas provided to the respiratory mask 120 of the user/ pilot. Also, the apparatus 130 alerts the user/ pilot if/ when the oxygen partial pressure falls below a warning level.

The user thereby becomes aware of the emerging dangerous situation involving hypoxia and could take any appropriate action, for example decreasing altitude, increasing the oxygen concentration of the breathing gas provided to the respiratory mask 120 and/ or activating an emergency oxygen reservoir.

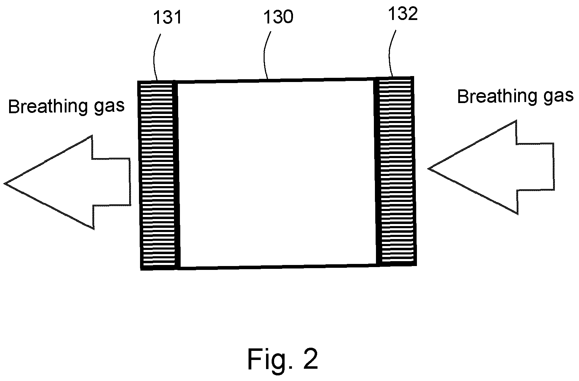

Figure 2 illustrates an apparatus 130 for monitoring the oxygen partial pressure in the breathing gas provided to the respiratory mask 120 of a user, for example a pilot. The apparatus 130 attaches to the breathing gas inlet 125 of the respiratory mask 120 and the breathing gas tubing 140 via attachment mechanisms 131, 132, ensuring a secure and reliable connection.

The first attachment mechanism 131 is situated on a first end section of the apparatus 130. The first attachment mechanism 131 is configured to attach the apparatus 130 to the breathing gas inlet 125 of the respiratory mask 120.

The second attachment mechanism 132 is situated on a second end section of the apparatus 130. The second end section of the apparatus 130 may be situated at an opposite side of the apparatus 130, in relation to the first end section thereof.

The apparatus 130, with the first attachment mechanism 131 and second attachment mechanism 132, provides a reliable and efficient solution for monitoring oxygen partial pressure in the breathing gas provided to the user.

The design of the attachment mechanisms 131, 132 also ensures that the apparatus 130 can be easily integrated into existing respiratory masks 120 and breathing gas tubings 140, enhancing the versatility and ease of use, without having to modify or change the respiratory mask 120, the breathing gas tubing 140, the pilot helmet 110 or any other previously existing equipment.

The apparatus 130 may be made in plastic for example some polymer (Polyethylene, Polypropylene, Polyvinyl Chloride, etc.), to keep weight low, yet efficiently enclosing the apparatus 130 and disallowing oxygen enriched air from the breathing gas tubings 140 to leak out in the cabin. However, aluminium/ aluminium alloy or titanium/ titanium alloy may be other options. In other embodiments, the apparatus 130 may be made in composite materials or for example comprise carbon fibre enhanced plastic. The material is selected to be compatible with high oxygen concentration while keeping weight low, decrease risks of mechanical failure or cracks and assuring isolation from leakage.

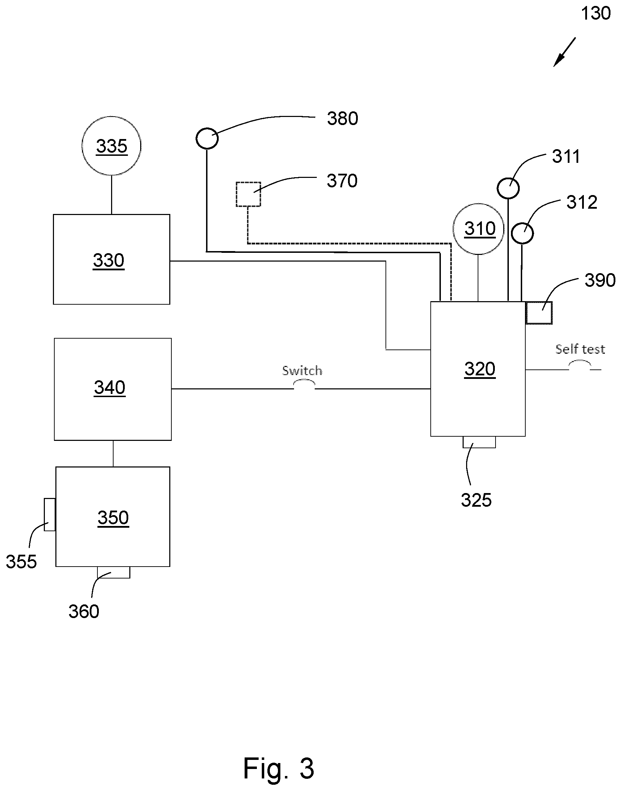

Figure 3 shows a schematic block diagram illustrating the components and connections of the apparatus 130 for monitoring oxygen partial pressure and alerting the user of imminent risk of hypoxia. The apparatus 130 comprises a sensor 310, a controller 320, a vibration actuator 335, and a power supply unit 340. The sensor 310, the vibration actuator 335, the controller 320 and the power supply unit 340 are maintained in one single unit. In some embodiments, the apparatus 130 also may comprise a visual output device 370 and/ or an olfactory emitter 380. The controller 320 is communicatively connected to the vibration actuator 335, and/ or to the visual output device 370 and/ or the olfactory emitter 380.

The sensor 310 is configured to continuously measure the oxygen partial pressure of the breathing gas provided to the user carrying the respiratory mask 120 and provide these measurements to the controller 320. The continuous measurements of the sensor 310 may be made iteratively at a configurable regular time interval. The continuous measurements of the sensor 310 may be made in real-time, i.e. at a frequency which is as intense as allowed by the capacity of the sensor 310 and the controller 320 respectively to handle and communicate the measurement data.

The configurable regular time interval may be every tenth of a second, every second, every five seconds, etc., in some non-limiting examples. A brief time interval between measurements ensures a fast detection of low oxygen partial pressure while a longer time interval saves power and thereby extends operating time of the power supply unit 340.

The sensor 310 is communicatively connected to the controller 320. The sensor 310, when having made the oxygen partial pressure measurement, provides the measurement to the controller 320. The controller 320 upon obtaining the measurement, compares the obtained oxygen partial pressure measurement with one or several pressure warning levels. When the oxygen partial pressure falls below a pressure warning level, the controller 320 triggers an alert via the vibration actuator 335. In some embodiments, the alert may also be triggered via the visual output device 370 and/ or via the olfactory emitter 380.

The controller 320 may comprise an integrated circuit, e.g. a System on Chip (SoC) and or a Microcontroller Unit (MCU), that integrates some, most or all components required for computational tasks. These components may comprise a central processing unit (CPU), memory interfaces, input/ output devices/ interfaces, flash storage, and secondary storage interfaces.

The vibration actuator 335 is configured to act on the respiratory mask 120 for generating a tactile alert. The vibration actuator 335 may for example comprise a vibration motor such as a coin vibration motor or a cylindrical vibration motor; or a Linear Resonant Actuator or similar vibration generating structure. The apparatus 130 may comprise a vibration actuator driver 330 in some embodiments.

The optional visual output device 370 may comprise a display, a diode, or a plurality of diodes arranged to emit light within the visible spectrum of the same or different colour/s. In case the visual output device 370 comprises a display, the oxygen partial pressure level may be output on the display.

In some embodiments, the apparatus 130 may comprise a battery charger 350, a wired battery charging interface 355, and/ or a wireless battery charging interface 360. Thereby, the power supply unit 340 when embodied as a rechargeable battery may be charged.

The power supply unit 340 powers the sensor 310, the controller 320, the vibration actuator 335 and possibly also various other components of the apparatus 130. The power supply unit 340 may ensure that the apparatus 130 operates independently without requiring any external power sources. The battery charger 350 and the battery charging interface 355 may facilitate the recharging of the power supply unit 340, in some embodiments. The battery charging interface 355 may connect the battery charger 350 to the power supply unit 340, allowing for efficient recharging. The optional battery charging interface 355 may comprise Universal Serial Bus (USB), USB-C, micro-USB, etc.

The battery charger 350 may optionally comprise a wireless charging interface 360 for inductive charging of the power supply unit 340.

The power supply unit 340 may alternatively comprise a power supply feed from the aircraft system, or from another apparatus-external source of electricity.

The apparatus 130 may also comprise a switch. The switch may control the power supply of the power supply unit 340 to the various components of the apparatus 130. The switch ensures that the apparatus 130 can be turned on or off as needed, providing an additional layer of control over the operation. In case of malfunction for example, it may be desired by the user to switch it off, for avoiding disturbance; or when grounded, for saving power.

The apparatus 130 may comprise an ambient pressure sensor 311 configured to measure ambient pressure and/ or an accelerometer 312 configured to measure acceleration.

The controller 320 may comprise a data logging interface 325, i.e. an external communication interface which may be wired or wireless in different embodiments.

Various measurement data, for example the oxygen partial pressure measurements of the sensor 310 utilised for the hypoxia alerting, and ambient pressure measurements of the ambient pressure sensor 311 and/ or the acceleration as measured by the accelerometer 312 may be stored in a memory 390 during operation. The ambient pressure measurements and/ or the acceleration, although not used for the hypoxia detection during operation may during later analysis provide information for improving future hypoxia detection, or for determining cause of various onboard incidents.

The data logging interface 325 allows the apparatus 130 to connect to external systems for data logging and further post-operation analysis.

The optional olfactory emitter 380 of some embodiments may, upon receiving an alert signal from the controller 320, may be configured to release a scent and/ or a restorative substance.

The restorative substance may comprise smelling salts and/ or ammonia, or a substance with similar reinvigorating effect. The ammonia/ smelling salts may irritate the nasal and lung membranes, triggering an inhalation reflex of the user that can momentarily improve oxygen intake. Thereby, a temporary boost in alertness is achieved, which may prevent the user from losing consciousness and/ or recover the user from a mild state of hypoxia and give time to manually release an emergency oxygen supply, or any other appropriate measure such as decreasing altitude.

The terminology used in the description of the embodiments as illustrated in the accompanying drawings is not intended to be limiting of the described system; structure; and/ or vehicle. Various changes, substitutions and/ or alterations may be made, without departing from the embodiments as defined by the appended claims. Elements of different embodiments may be combined with each other, thereby achieving additional advantages.

As used herein, the term "and/ or" comprises any and all combinations of one or more of the associated listed items. The term "or" as used herein, is to be interpreted as a mathematical OR, i.e., as an inclusive disjunction; not as a mathematical exclusive OR (XOR), unless expressly stated otherwise. In addition, the singular forms "a", "an" and "the" are to be interpreted as "at least one", thus also possibly comprising a plurality of entities of the same kind, unless expressly stated otherwise. It will be further understood that the terms "includes", "comprises", "including" and/ or "comprising", specifies the presence of stated features, actions, integers, steps, operations, elements, and/ or components, but do not preclude the presence or addition of one or more other features, actions, integers, steps, operations, elements, components, and/ or groups thereof. A single unit such as e.g., a structural element may fulfil the functions of several items recited in the claims. The mere fact that certain measures are recited in mutually different dependent claims does not indicate that a combination of these measures cannot be used to advantage.

## Claims

1. An apparatus (130) for monitoring level of oxygen partial pressure and alerting a user of imminent risk of hypoxia, comprising:
a first attachment mechanism (131) configured to attach the apparatus (130) to breathing gas inlet (125) of a respiratory mask (120) of the user; and
a second attachment mechanism (132) configured to attach the apparatus (130) to a breathing gas tubing (140) providing oxygen-enriched air from an onboard oxygen generation system to the respiratory mask (120);
a sensor (310) configured to measure the oxygen partial pressure in the breathing gas tubing (140);
a vibration actuator (335), situated in a position abutting a part of the respiratory mask (120) or in close vicinity to the respiratory mask (120), and configured to provide a tactile alert causing vibrations on the respiratory mask (120) when activated;
a controller (320), communicatively connected to the sensor (310) and the vibration actuator (335), configured to:
obtain a measurement of the oxygen partial pressure from the sensor (310);
compare the obtained measurement with a pressure warning level; and
trigger an alert via the vibration actuator (335) when the oxygen partial pressure is lower than the warning level; and
a power supply unit (340) configured to power the sensor (310), the vibration actuator (335) and the controller (320); and
wherein the sensor (310), the vibration actuator (335), the controller (320) and the power supply unit (340) are maintained in one single unit, separate from, although attachable to, the respiratory mask (120) and the breathing gas tubing (140) via the respective attachment mechanisms (131, 132).

2. The apparatus (130) according to claim 1, comprising a visual output device (370) communicatively connected to the controller (320), wherein the visual output device (370) is configured to provide a visual alert when activated; and wherein the controller (320) is configured to trigger the alert also via the visual output device (370) when the oxygen partial pressure is lower than the warning level.

3. The apparatus (130) according to any one of the preceding claims, wherein the controller (320) is configured to compare the obtained measurement with a plurality of pressure warning levels and trigger different types of alerts, based on the outcome of the comparison.

4. The apparatus (130) according to any one of the preceding claims, wherein the controller (320) is configured to trigger escalating alerts in response to decreasing oxygen partial pressure levels.

5. The apparatus (130) according to claim 4, wherein the escalating alerts comprises an increase in vibration amplitude and/ or vibration intensity of the vibration actuator (335) and/ or variation in emitted colour and/ or increased blinking intensity of the visual output device (370).

6. The apparatus (130) according to any one of the preceding claims, wherein any pressure warning level is adaptable to an individual hypoxia sensibility of the user.

7. The apparatus (130) according to any one of the preceding claims, wherein the power supply unit (340) comprises a rechargeable battery.

8. The apparatus (130) according to any one of the preceding claims, wherein the power supply unit (340) comprises a wirelessly rechargeable battery.

9. The apparatus (130) according to any one of the preceding claims, further comprising a memory (390) configured to store measurement data during operation; and a data logging interface (325) configured to export collected measurement data from the memory (390), for post-operation analysis.

10. The apparatus (130) according to claim 9, comprising an ambient pressure sensor (311) configured to measure ambient pressure and/ or an accelerometer (312) configured to measure acceleration; and wherein the measurement data stored by the memory (390) and exported for post-operation analysis comprises the measured ambient pressure and/ or acceleration.

11. The apparatus (130) according to any one of the preceding claims, wherein the controller (320) is configured to run a self-test program for confirm correct apparatus functionality and power supply status.

12. The apparatus (130) according to any one of the preceding claims, comprising an olfactory emitter (380) communicatively connected to the controller (320), wherein the olfactory emitter (380) is configured to provide an olfactory alert when activated; and wherein the controller (320) is configured to trigger the alert also via the olfactory emitter (380) when the oxygen partial pressure is lower than the warning level.
